# EUROPEAN PATENT APPLICATION

(11) **EP 3 738 556 A1**
(43) Date of publication of application: **18.11.2020**
(21) Application number: 19174928.2
(22) Date of filing: 16.05.2019
(51) Int. Cl.: A61F 9/00

(54) **INSERTION DEVICE FOR INSERTING DEFORMABLE PROSTHESIS INTO AN EYE**

(71) Applicant: Ecole Polytechnique Fédérale de Lausanne EPFL-TTO, 1015 Lausanne (CH); Fondation Asile Des Aveugles, 1002 Lausanne (CH)
(72) Inventor: GHEZZI, Diego, 1202 Geneve (CH); WOLFENSBERGER, Thomas J., 1002 Lausanne (CH); VILA, Charles-Henri, 1202 Geneve (CH)
(74) Representative: Morabito, Sara

(57) **Abstract**

An insertion device (100; 100') for inserting a retinal prosthesis (200) into the eye of a patient comprises: a handle portion (1; 1') arranged to be grasped by a user of the insertion device (100; 100'), a cannula element (12; 12') arranged to be inserted into the eye of a patient positioned at a longitudinal end (4A) of the handle portion (1; 1'), a holding portion (3; 3') arranged for holding the retinal prosthesis (200) in the coiled configuration (P), the holding portion (3; 3') being provided with an internal longitudinal hole (24; 24') so dimensioned to house the retinal prosthesis (200) in the coiled configuration (P) and to avoid the retinal prosthesis (200) to uncoil in the insertion device (100; 100'), an actuating mechanism (2, 21; 2', 32') coupled to the handling portion (1; 1') and arranged for being rotated for moving the retinal prosthesis (200) in relation to the cannula element (12; 12') for inserting the retinal prosthesis (200) into the eye (300) of the patient, wherein the insertion device (100; 100') further comprises a conversion mechanism for transforming the rotation movement of the actuating mechanism (2, 21; 2', 32') in a translation movement for moving the retinal prosthesis (200) in relation to the cannula element (12; 12').

## Description

The invention relates to an insertion device for inserting a deformable prosthesis in an eye of a patient.

In particular, the invention relates to an insertion device for inserting large retinal prostheses into the eye of the patient.

In presence of some diseases, such as for example *retinitis pigmentosa*, it is necessary to substitute the retinal photoreceptors of a patient implanting into the eye of the patient a retinal prosthesis.

The implantation of retinal prostheses is considered to be a relatively safe procedure.

The retinal prostheses are inserted through an incision effected on the surface of the cornea or the sclera of the eye.

Nevertheless, one of the principal disadvantages of conventional insertion devices is that they require a relatively large incision in the ocular tissue or, on the contrary, that only prostheses having reduced dimensions could be implanted.

It is necessary to limit the dimension of the incisions in order to avoid post-operative complications.

It has been therefore suggested to use a deformable lens, which have memory characteristics enabling the lens to be deformed to fit through a smaller ocular incision and then re-deformed once in the eye to take the final configuration.

It has been proposed by EP0270257 a device for inserting an intraocular deformable lens 130 into the eye of the patient, which tries to solve the above indicated drawback.

The device of EP0270257, comprises an insertion cannula 112 having a cannula head 114 and a cannula portion 116.

The cannula head 114 defines an inner loading chamber 118 including a cylindrical section 118a and a conical section 118b in communication with the cylindrical section 118a. The conical section 118b converges towards and protrudes into the cannula member 116.

The device of EP0270257 further comprises a tubular body 102 internally hollow arranged to be connected by means of an overclamping nut 120 to the insertion cannula 112, i.e. the insertion cannula 112 is inserted into the overclamping nut 120 which is in turn screwed onto the tubular body 120.

The device of EP0270257 further comprises a soft elastomeric plunger 126 arranged to be inserted into one end of the tubular body 102, opposite to the end at which the insertion cannula 112 is connected and to move the intraocular lens 130 through the loading chamber 118. The plunger 126 is provided with a screw 128, so that, as the screw 128 is turned, the plunger 126 is forced towards the cannula member 116 and the lens 130 is pressed against the inner surfaces of the conical section 118b which thereby exert a radial force against the lens 130. As a consequence, the lens 130 is deformed into its extruding position in the cannula member 116.

This movement deforms the lens 130 reducing the cross-sectional area thereof, so that the lens can be delivered into the cannula member.

When the lens is fully loaded into the cannula 130 the plunger 126 is removed from the tubular body 102 and a push rod 134 is inserted therein.

The push rod 134 includes an elongated rod 136 configured to fit into the cannula member 116 and a tip 138 made of soft material. The push rod 134 is driven by a screw 139 towards the distal tip 140 of the cannula member 116 so as to push the lens 130 out from the cannula member 116 and into the eye by twisting its screw member end 139.

Even if such a device can be used with a deformable lens, it has nonetheless some drawbacks.

The lens 130 is deformed during its movement through the device and the risk exists that the lens is damaged. In the device of EP0270257 it is provided for using a plunger 126 made of soft material and a push rod having a tip of soft material for trying to reduce the damages to the lens. This could nonetheless have the effect that the pushing action is not effective. Nonetheless the lens 130 risks to be damaged due to the friction against the wall of the device.

Additionally, the lens is positioned in un-deformed configuration in the conical section 118b of the chamber 118 and it is deformed in the movement along towards the cannula. Therefore, the risk exists that the lens is not deformed in the desired manner. This can also have the effect that the lens cannot be positioned in the correct position inside the eye.

Additionally, the device of EP0270257 has many different parts to be assembled together and this makes the assembling and functioning of the device most complicated.

Moreover, it is necessary to pay a lot of attention in moving the plunger 126 so that the lens is deformed in the correct way and to avoid that the lens is deformed in a position in which it cannot be correctly deployed in the eye. Therefore, a certain skill is required for correctly using the device of EP0270257.

An object of the invention is to provide an insertion device configured to overcome the drawbacks of the known device.

Another aim of the invention is to provide a device which is structurally and functionally designed for overcoming the drawbacks of the prior art.

Another object of the invention is to provide a device allowing a retinal prosthesis even of large dimensions to be implanted minimising the incision to be made on the sclera (or of the cornea) of the patient.

According to an aspect of the invention it is provided an insertion device for inserting a retinal prosthesis into the eye of a patient comprising: a handle portion arranged to be grasped by a user of the insertion device; a cannula element arranged to be inserted into the eye of a patient and positioned at a longitudinal end of the handle portion, a holding portion arranged for holding the retinal prosthesis in the coiled configuration, the holding portion being provided with an internal longitudinal hole so dimensioned to house the retinal prosthesis in the coiled configuration and to avoid the retinal prosthesis to uncoil in the insertion device, an actuating mechanism coupled to the handling portion and arranged for being rotated for translating the retinal prosthesis in relation to the cannula element for inserting the retinal prosthesis into the eye of the patient, wherein the insertion device further comprises a conversion mechanism for converting the rotation movement of the actuating mechanism in a translation movement for translating the retinal prosthesis in relation to the cannula element in the coiled configuration.

Owing to this aspect of the invention, it is obtained a device allowing a retinal prosthesis of larger dimension than usual ones to be inserted into the eye of the patient without increasing at the same time the invasiveness of the insertion device.

Owing to the configuration of the internal longitudinal hole, it is avoided that the retinal prosthesis uncoils in the insertion device. Therefore, the retinal prosthesis is not deformed during its movement along the insertion device.

This assures that the retinal prosthesis is delivered in the correct position in the eye of the patient.

The retinal prosthesis is coiled along a coiling axis and it is, then, inserted in the holding portion of the insertion device in the coiled configuration.

The retinal prosthesis in the coiled configuration is so positioned in the longitudinal hole of the holding portion so that the spiral of the coiled retinal prosthesis is perpendicular to the longitudinal axis of the longitudinal hole.

In a version, the handle portion is provided with a longitudinal cavity and the actuating mechanism comprises a translating device for translating the holding portion along the longitudinal cavity in relation to the cannula element for inserting the retinal prosthesis into the eye of the patient.

In this version the retinal prosthesis is carried by the holding portion along the longitudinal cavity and outside from the insertion device. The holding portion does not translate in relation to the retinal prosthesis in the movement of the holding portion in the longitudinal cavity for inserting the retinal prosthesis into the eye of the patient.

This assures that the retinal prosthesis is not subjected to a friction action and thus that it is not damaged during the translation into the insertion device before being inserted into the eye of the patient.

There is no relative movement along the translation in the longitudinal cavity between the retinal prosthesis and the holding portion, and the retinal prosthesis is thus protected.

In a version, the translating element is so configured that the holding portion is slideable in the longitudinal cavity in relation to the cannula element between a retracted configuration in which the holding portion is positioned inside the cannula element and an extended configuration in which the holding portion is positioned outside the cannula and the retinal prosthesis may be delivered outside the insertion device into the eye of the patient.

In this way, the holding portion holds the retinal prosthesis during its movement along the longitudinal cavity and carries the retinal prosthesis along the longitudinal cavity, and any relative movement between the holding portion and the retinal prosthesis is avoided. Therefore, the retinal prosthesis is not damaged or deformed in the translation along the insertion device.

In a version, the insertion device is further provided with a guide device for guiding the translation of the translation device into the insertion device along the longitudinal cavity thereof.

The guide device comprises at least one groove defined in the wall of the longitudinal cavity and extending along the longitudinal axis thereof and configured to avoid the rotation of the translation device in its movement along the insertion device about the longitudinal axis of the longitudinal cavity.

Advantageously, the translation device is provided with at least one protruding portion arranged to match with the at least one groove so as to be slideably housed in the groove for cooperating with the groove for guiding the movement of the translation device. Advantageously, the guide device comprises a pair of grooves defined at diametrically opposing position in the longitudinal hole and a pair or diametrically opposed protrusion in the translation device.

In this way, no rotation of the translation device in the longitudinal hole is allowed.

The guide device may be advantageously provided with a stop element for limiting the stroke of the translating device in the longitudinal cavity.

The holding portion advantageously defines a holding cavity in which the retinal prosthesis is housed in its coiled configuration and keeps the retinal prosthesis in the coiled configuration and avoids an unwanted uncoiling of the retinal prosthesis in the insertion device.

In a version, the holding portion is provided with at least two holding elements, the retinal prosthesis being inserted and held between the holding elements in the coiled configuration. Advantageously, the retinal prosthesis in the coiled configuration is so positioned in the holding portion that the holding elements extends substantially parallel to the coiling axis of the retinal prosthesis in the coiled configuration.

Advantageously, the holding elements are interposed between the retinal prosthesis and the wall of the longitudinal cavity, so that the holding elements protect the retinal prosthesis during the movement along the insertion device.

This further allows that the holding elements keep the retinal prosthesis firmly in the insertion device.

This also increases the insertion precision obtainable with the device of the invention

In a version, the holding elements comprises two prongs extending along a longitudinal axis of the holding portion and mutually facing, the retinal prosthesis in the coiled configuration being inserted between the two prongs.

Advantageously, the holding elements are made of a flexible material so as to be deformable to help the delivery of the retinal prosthesis outside from the insertion device.

The holding elements can be for example made of plastics, such as polypropylene, polycarbonate, in particular Accura 60 may be used.

Nonetheless, many different plastic materials suitable for being used in the ophthalmic field could be used.

The holding elements are made of a plastic material having a certain flexibility so they can be easily deformed for inserting the retinal prosthesis there between and for allowing the retinal prosthesis to be ejected from the holding elements into the eye of the patient.

The holding elements can be made by membranes extending from the holding portion.

In another version of the insertion device, the holding portion is fixed in relation to the handle portion, i.e. the holding portion is not translatable in relation to the handle portion and the retinal prosthesis translates along the internal longitudinal hole in relation to the cannula element and to the holding portion for being inserted into the eye of the patient.

The holding portion is fixed in relation to the cannula element.

In this version, the holding portion is defined in a base portion of the cannula element, the base portion being provided with a tubular element arranged within the base and the cannula element and defining the holding portion in which the retinal prosthesis in the coiled configuration is inserted and moved.

The tubular element is advantageously concentric with the base and the cannula element. The base portion is provided with connecting means for being connected to the handle portion so as to connect the cannula element to the handle portion of the insertion device.

In this version, it is further provided an actuating mechanism comprising a pushing element for pushing the retinal prosthesis outside the holding portion. The pushing element is arranged for interacting with almost all the edge of the retinal prosthesis in the coiled configuration pushing the retinal prosthesis in a uniform manner along the tubular element. The pushing element interacts with almost all the spires of the retinal prosthesis in the coiled configuration. This assures that the retinal prosthesis is delivered in the correct position into the eye of a patient.

The features and advantages of the invention will be better appreciated from the detailed description of some preferred embodiments thereof, illustrated by way of non-limiting example with reference to the appended drawings, in which:
- Fig. 1 is a perspective view of a first version of an insertion device according to the invention;
- Fig. 2 is a sectional view of the insertion device of Fig. 1;
- Fig. 3 is a sectional interrupted view of a handle portion of the insertion device of Fig. 1;
- Fig. 4 is a perspective interrupted view of a holding portion of the insertion device of Fig. 1;
- Fig. 5 is a detail of Fig. 4;
- Fig. 6 is a perspective view of the actuating mechanism of the insertion device of Fig. 1;
- Fig. 7-9 are perspective interrupted views of the insertion device of Fig. 1 in three different operating configurations;
- Fig. 9A is a schematic interrupted view of the insertion device of Fig. 1 in another operating configuration;
- Fig. 9B is a view of a pushing element;
- Fig. 10 is a sectional view of a second embodiment of the insertion device according to the invention;
- Fig. 11 is an interrupted sectional view of a handle portion of the insertion device of Fig. 10;
- Fig. 12 is a lateral view of a plunger portion of the insertion device of Fig. 10;
- Fig. 13 is a perspective view of the holding portion of the insertion device of Fig. 10;
- Fig. 13A is a detail of Fig. 13
- Fig. 14 is a sectional view of the holding portion of Fig. 13;
- Fig. 15 is a detail of the holding portion of Fig. 13;
- Fig. 16 is a schematic view showing the ocular incision for the device of the invention;
- Fig. 17 is a schematic view showing the insertion of the device of the invention into the eye;
- Fig. 18 is a schematic view showing a retinal prosthesis in a coiled configuration;

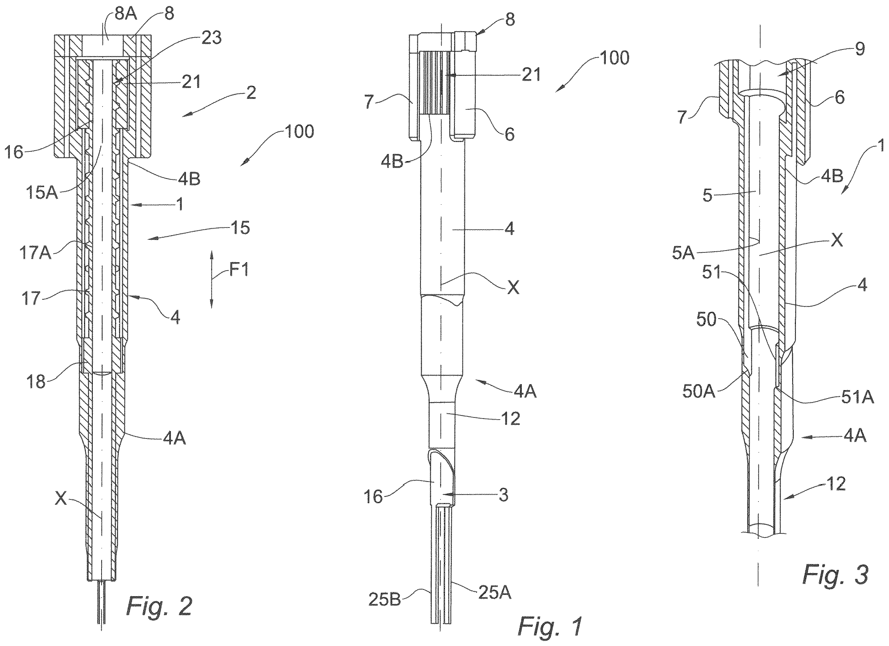

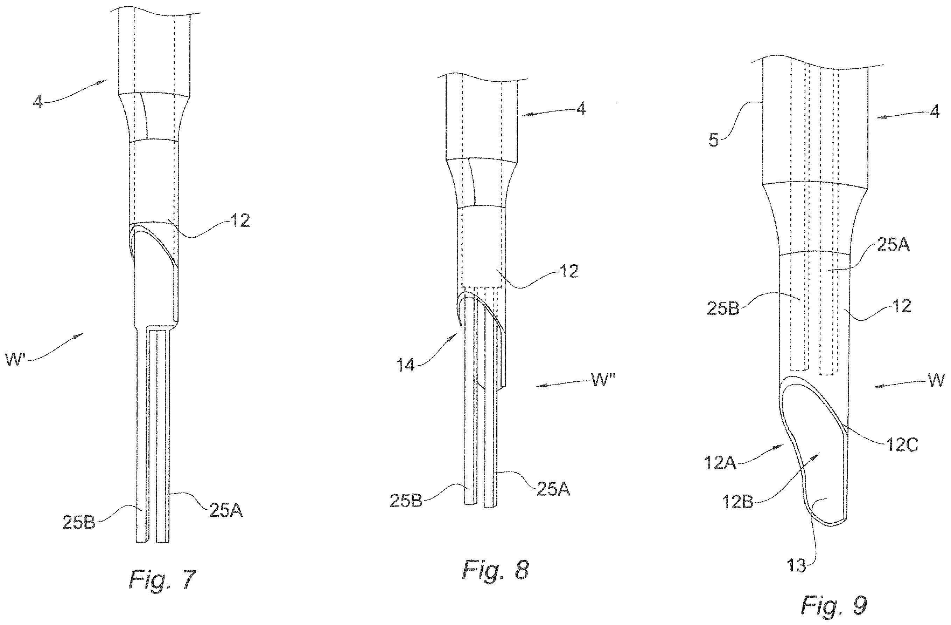

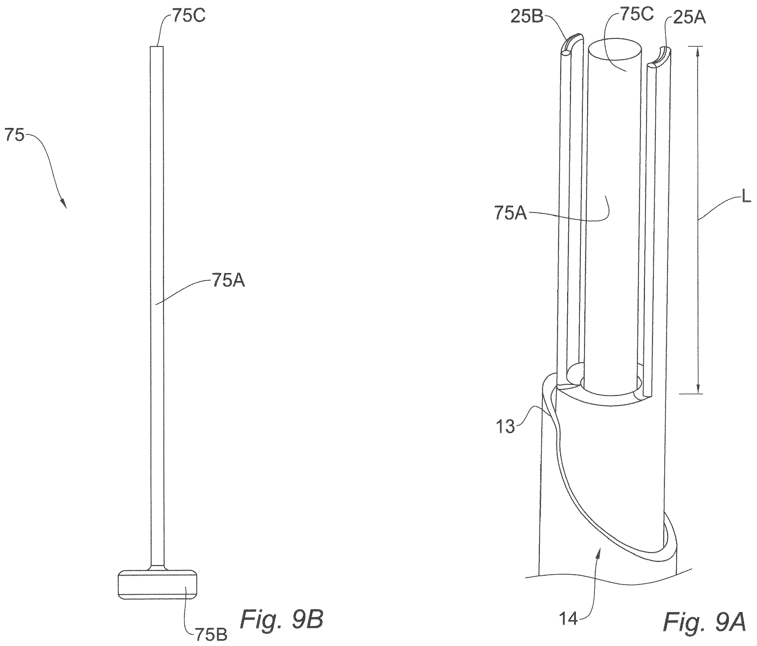

With reference to the Figs. 1-9 and 9A it is shown a first embodiment of the insertion device 100 according to the invention.

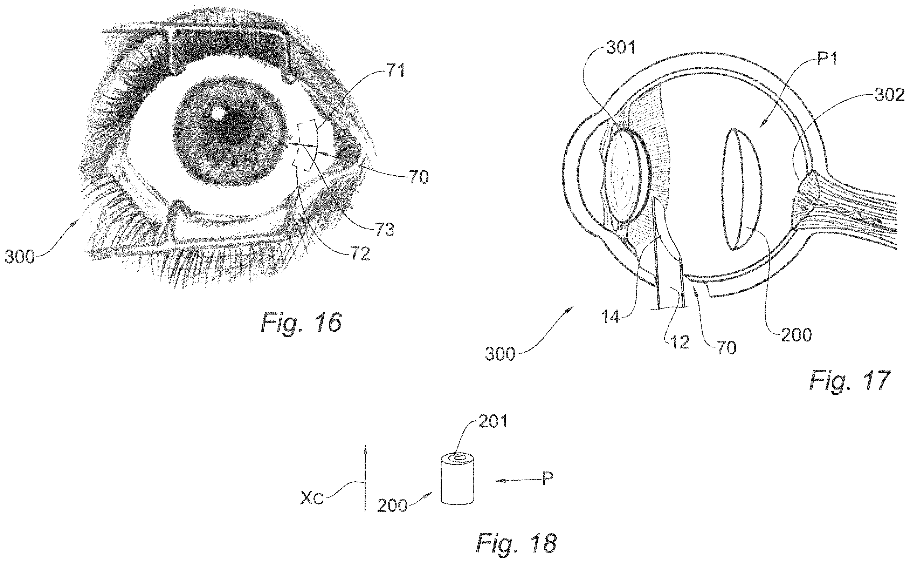

The insertion device 100 is arranged for inserting a retinal prosthesis 200, better visible in Fig. 18, into the eye 300 of the patient and it is particularly suited for inserting a retinal prosthesis having large dimension into the eye of the patient.

The insertion device 100 is particularly suitable for inserting retinal prosthesis having dimension bigger than usual retinal prosthesis, in particular, it is configured for inserting retinal prosthesis having a length of more than 7 mm.

Usually retinal prosthesis of less than 5 mm in size are used. The retinal prosthesis 200 is usually made of soft material having memory characteristics and it has a shape resembling the curvature of the eye. The retinal prosthesis 200 has the shape of a spherical cap.

The retinal prosthesis 200 is coiled around a coiling axis Xc, as better visible in Fig. 18, so that the edge of the retinal prosthesis 200 forms a spiral 201 that lies in a plane that is substantially perpendicular to the coiling axis Xc and so as to conform the retinal prosthesis 200 in a coiled configuration P.

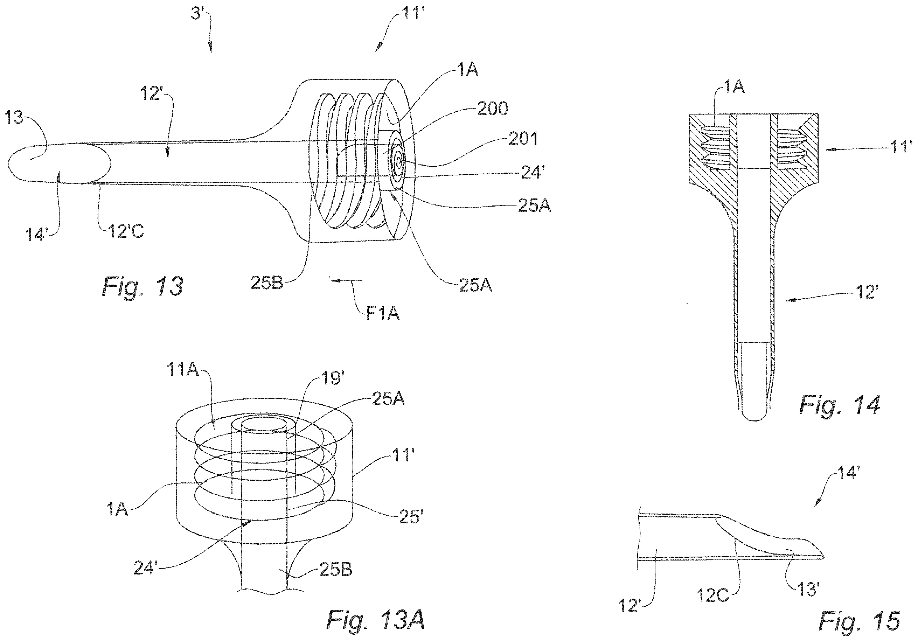

The retinal prosthesis 200 is loaded in the holding portion 3 of the insertion device 100 in the coiled configuration P, as visible in better detail in Fig. 18 and 13.

The retinal prosthesis 200 is translated in the insertion device in the coiled configuration P and it is delivered in the eye of the patient in the coiled configuration P. When the retinal prosthesis 200 is delivered by the insertion device 100 and positioned in the eye 300 of the patient, the retinal prosthesis 200 spontaneously de-coils so that is takes on the use configuration P1, visible in Fig. 17, in which the retinal prosthesis 200 is deployed and has the curvature of the eye.

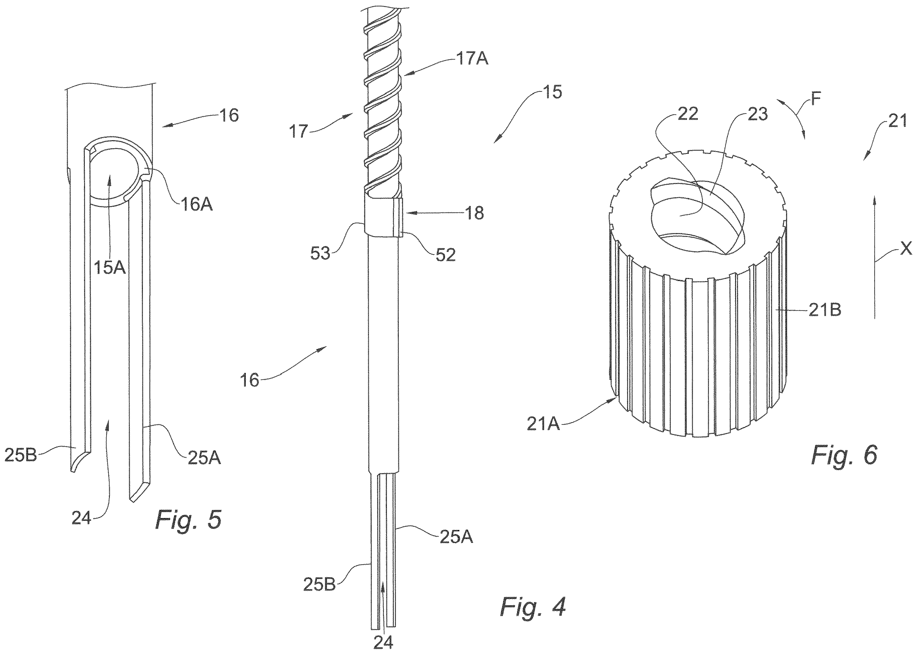

The insertion device 100 comprises a handle portion 1 arranged to be grasped by a user of the insertion device 100 shown in greater detail in Figs. 1-3, an actuating mechanism 2 coupled to the handling portion 1 for moving the retinal prosthesis 200 in the insertion device 100 and a holding portion 3 arranged for holding the retinal prosthesis 200 in the coiled configuration P, shown in better detail in Figs. 4 and 5.

The handle portion 1 comprises a substantially tubular body 4 delimited by an external surface S and internally hollow so as to define a longitudinal cavity 5 extending along a longitudinal axis X of the tubular body 4 for moving the retinal prosthesis 200 along the insertion device 100.

The longitudinal cavity 5, visible in better detail in Fig. 5, is delimited by a wall 5A that is provided with a first and a second groove 50, 51 extending along the longitudinal axis X of the longitudinal cavity 5 and arranged for guiding the movement of the retinal prosthesis 200 in the insertion device 100 for inserting the retinal prosthesis 200 in the eye of a patient.

The first and the second grooves 50, 51 are positioned on diametrically opposing positions. The first and the second grooves 50, 51 act as guide device for guiding the movement of the retinal prosthesis 200 along the insertion device 100.

The provision of a guide device allows a more precise movement of the retinal prosthesis 200 in the insertion device 100 to be obtained.

The first and the second grooves 50, 51 are so shaped to house corresponding protrusions 52-53 in the holding portion 3 for guiding the movement of the retinal prosthesis 200 into the insertion device 100, as better explained in the followings. The first and the second grooves 50, 51 are so shaped that the corresponding protrusions 52-53, shown in Figure 6, may slide along respectively the first and the second groove 50, 51 during use of the insertion device 100.

In another version not shown, a different number of grooves may be provided, preferably equally spaced along the circumference of the wall 5A of the longitudinal cavity 5.

In another version not shown, the insertion device 100 comprises a different guide device for guiding the movement of the retinal prosthesis 200 along the insertion device 100.

The guide device is provided with at least one stroke limiting element for limiting the translation of the holding 3 portion in the longitudinal cavity 5.

In the version shown, the first and the second grooves 50, 51 are provided with corresponding stop element 50A, 51A arranged for stopping the translation of the holding portion 3, as better described in the followings.

The tubular body 4 is provided at a first longitudinal end 4A thereof with a cannula element 12 arranged for being inserted into the eye 300 of a patient.

The cannula 12 is internally hollow and defines an ejection cavity 120 through which the retinal prosthesis 200 is delivered for being inserted into the eye 300 of the patient.

The cannula 12 is coaxial with the tubular body 4 so that the longitudinal axis of the cannula 12 and of the ejection cavity 12A is parallel with and corresponding with the longitudinal axis X of the longitudinal cavity 5. The cannula 12 is provided with a mouth 14 through which the retinal prosthesis 200 is injected into the eye.

The mouth 14 is bevelled, i.e. the edges 12C of the mouth 14 are inclined in relation to the longitudinal axis X of the cannula 12, so that a mouth 14 having the shape of a flute-tip is defined.

Owing to this configuration, a wall portion 13 of the wall 12A of the cannula 12 faces the mouth 14 and acts as a guide element for the retinal prosthesis 200 flowing out from the cannula 12. Therefore, the wall 13 guides the ejection from the cannula 12 of the retinal prosthesis 200.

In addition, the wall portion 13 allows the ejection direction of the retinal prosthesis 200 to be adjusted and defined, avoiding the retinal prosthesis 200 to be ejected from the cannula 12 in the wrong direction and/or wrong position into the eye.

This also allows the eye 300 of the patient to be protected.

The flute-tip shape of the mouth 14 protects the lens 301 of the eye 300: the insertion device 100 can be so oriented that the wall 13 of the cannula 12 is closest to and tangent to the lens 301 of the eye, as visible in Fig. 17, so that the mouth 14 faces the retina 302.

In this way, once the retinal prosthesis 200 is pushed out from the mouth 14 it is ejected towards the retina 302 of the eye rather than towards the lens 301 of the eye 300.

In other version not shown, the cannula is provided with a mouth having a different shape; the form of the mouth of the cannula should be chosen so as to allow the gradual release of the retinal prosthesis into the eye. A cannula having an alligator type mouth could be for example employed.

The cannula 12 has reduced thickness so as to minimize the invasiveness of the device of the invention and, on the other side to maximize the space free for the passage of the retinal prosthesis.

Advantageously, the cannula 12 has a thickness comprised between 100-250µm, the ejection cavity 120 has a diameter comprised between 2-5 mm.

The handle portion 1 further comprises two arms 6, 7, extending from a second longitudinal end 4B of the tubular body 4 that is opposite to the first longitudinal end 4A in a direction substantially parallel to the longitudinal axis X of the tubular body 4. The handle portion 1 further comprises a covering portion 8 fixed at the free ends of the arms 6, 7, i.e. opposite to the tubular body 4.

In this way, the covering portion 8 and the arms 6, 7 define in the handle portion 1 an actuating cavity 9 in which the actuating mechanism 2 can be housed, as explained in the followings. The covering portion 8 is provided with a through hole 8A in the longitudinal direction so as to allow the holding portion 3 to be inserted into the insertion device 100, as better explained in the following.

The actuating mechanism 2 comprises a nut 21, better visible in Fig. 6, arranged for being rotated by the user for moving the retinal prosthesis 200 in relation to the cannula element 12 for inserting the retinal prosthesis 200 into the eye 300 of the patient, as explained in the followings.

The nut 21 has an internal threaded hole 22 that, when the nut 21 is inserted into the actuating cavity 9, is coaxial with the longitudinal cavity 5 of the tubular body 4.

The external wall 21A of the nut 21 is provided with gripping means 21B for improving the grip of the nut 21 from a user of the insertion device 100 and for improving the precision of the actuation of the nut 21.

In the version shown, the external wall 21A is knurled so as to provide a better grip to the user. In the version shown, the knurling is formed by a plurality of grooves and protrusion extending along the longitudinal axis of the nut and alternating one with another and forming the gripping means 21B of the nut 21.

In other versions, different shapes of knurl could be provided.

In other versions, different kinds of gripping means could be also used.

The nut 21 is dimensioned for being rotatably inserted in the actuating cavity 9, so that when the nut 21 is inserted in the actuating cavity 9, it can be rotated about a longitudinal axis X of the longitudinal cavity 5 for moving the retinal prosthesis 200 in relation to the cannula element 12, as explained in the following.

The nut 21 is rotatable about the longitudinal axis X as indicated by the arrow F in Fig. 5 for moving the retinal prosthesis 200 in relation to the cannula element 12.

The actuating cavity 9 and the nut 21 are, on the contrary, configured to avoid any axial translation of the nut 21 in relation to the actuating cavity 9 when the nut 21 is inserted into the actuating cavity 9.

The insertion device 100 further comprises a translation device 15, better visible in Figs. 4 and 5, for translating the retinal prosthesis 200 outside the cannula element 12.

The translation device 15 comprises a body 16 that is arranged to be slideably inserted into the longitudinal cavity 5 so as to be slideable inside the longitudinal cavity 5 along the longitudinal axis X thereof, as better explained in the followings.

The translation device 15 is so configured that when it is inserted into the longitudinal cavity 5 the body 16 is coaxial with the longitudinal cavity 5 so that the longitudinal axis of the body 16 corresponds to the longitudinal axis X of the longitudinal cavity 5.

The translation device 15 comprises a threaded portion 17, a guide portion 18 and the holding portion 3 that are positioned one after another along the longitudinal axis X of the body 16.

The guide portion 18 is interposed between the threaded portion 17 and the holding portion 3 along the longitudinal axis X of the body 16.

The threaded portion 17 is provided with an external thread 17A shaped to be coupled with the internal thread 23 defined in the nut 21 for moving the translation device 15 along the longitudinal cavity 5.

The guide portion 18 is arranged for guiding the movement of the translation device 15 along the longitudinal cavity 5.

The guide portion 18 is provided with two protrusions 52-53 configured to match with the grooves 50-51 provided in the longitudinal cavity 5, so that the two protrusions 52-53 may slide along the grooves 50-51.

As indicated above, the provision of the grooves and protrusions respectively in the handle portion 1 and in the translation device 15, i.e. in the holding portion 3, avoids any rotation of the translation device 15 about the longitudinal axis X of the longitudinal cavity 5.

The protrusions 52, 53 are provided on diametrically opposing positions on the guiding portion 18. As indicated above, a different number of grooves/protrusions can be provided in the insertion device of the invention.

Analogously, in another version not shown the protrusion(s) could be provided in the longitudinal cavity 5, the translation device 15 being provided with corresponding grooves configured to match with the protrusion to allow the translation device 15 to be translated along the longitudinal cavity 5.

In other versions, the insertion device could be provided with a different guide device configured to allow the translation device 15 to be translated along the longitudinal cavity 5 and to avoid the rotation of the translation device 15 about the longitudinal axis X.

The holding portion 3 of the translation device 15 is arranged for holding the retinal prosthesis 200 in the coiled configuration P.

The holding portion 3 is provided with an internal longitudinal hole 24 so dimensioned to house the retinal prosthesis 200 in the coiled configuration P and to avoid the retinal prosthesis 200 to uncoil in the insertion device 100, i.e. to take the deployed configuration P1. The internal longitudinal hole 24 is so configured to avoid a partial uncoiling of the retinal prosthesis 200 from the coiled configuration P.

The holding portion 3 is so configured that the internal longitudinal hole 24 has a longitudinal axis X that is corresponding to the longitudinal axis X of the longitudinal cavity 5. The longitudinal hole of the holding portion has a diameter of about 1-2mm.

The retinal prosthesis 200 is coiled along the coiling axis Xc to take on the coiled configuration P and it is then inserted in the longitudinal hole 24 of the holding portion 3 in the coiled configuration P.

The retinal prosthesis 200 in the coiled configuration P is so positioned in the longitudinal hole 24 that the spiral 201 of the coiled retina P prosthesis is perpendicular to the longitudinal axis X of the longitudinal hole 24.

The holding portion 3 comprises at least a holding element for holding the retinal prosthesis 200 in the coiled configuration P.

In the version shown, the holding portion 3 comprises two prongs 25A, 25B acting as holding elements of the insertion device 100 and extending along the longitudinal axis X and positioned at diametrically opposing positions in the holding portion 3.

The prongs 25A, 25B define the longitudinal hole 24 inside which the retinal prosthesis 200 in the coiled configuration P is positioned and translated along the insertion device 100.

The prongs 25A, 25B extend from a longitudinal end 16A of the body 16 and extend along the longitudinal axis X of the holding portion 3 and are mutually facing so that the retinal prosthesis 200 is inserted therebetween in the coiled configuration P.

Advantageously the prongs have a length "L" that is equal or at least slightly longer that the length of the retinal prosthesis 200 in the coiled configuration so that the retinal prosthesis 200 is held by the prongs in its entire length.

This assures that the retinal prosthesis 200 is protected.

The prongs 25A, 25B are made of a flexible material, such as polypropylene, polycarbonate, in particular Accura 60. Any other plastic material suitable for use in the filed can be used. The prongs 25A, 25B are so configured so as to be deformable to help the delivery of the retinal prosthesis outside from the insertion device 100, as better explained in the followings. The provision of the two prongs 25A, 25B allows the retinal prosthesis 200 to be carried along the insertion device 100 before being injected into the eye and then to be delivered into the eye.

The prongs 25A, 25B act as holding device for the retinal prosthesis 200 holding the retina in the coiled configuration P avoiding the uncoiling of the retinal prosthesis 200, as carrying device carrying the retinal prosthesis 200 through the longitudinal cavity and also as delivering device allowing the retinal prosthesis 200 to be delivered into the eye.

This reduces and avoids any damage to the retinal prosthesis 200.

The edges of the prongs 25A, 25B are smoothed and rounded to as to minimize the trauma in case of contact with any ocular surfaces.

Additionally, the prongs 25A, 25B improves he loading of the retinal prosthesis 200 into the insertion device 100.

The prongs 25A, 25B may act like tweezers so as to ensure that the retinal prosthesis 200 in a coiled configuration P can be "picked up" and retracted inside the insertion device 100 maintaining the coiled configuration P.

In addition, the prongs 25A, 25B are flexible and yet rigid enough to allow manipulation of the retinal prosthesis 200 in a tweezer like fashion.

These advantages can be obtained without damaging the retinal prosthesis 200.

As indicated above, retinal prosthesis 200 is inserted in the longitudinal hole 24 defined by the prongs 25A, 25B, the latter being so positioned to be interposed in use between the retinal prosthesis 200 and the wall 5A of the longitudinal cavity 5, so that the holding elements protect the retinal prosthesis 200 during the movement along the insertion device 100 to be inserted into the eye of the patient.

The holding portion 3 is slideable in the longitudinal cavity 5 as indicated by the translation arrow F1 in Fig. 2 in relation to the cannula element 12 between a retracted configuration W, shown in Fig. 9, in which the holding portion 3 and the prongs 25A, 25B are positioned inside the cannula element 12, an extended configuration W', shown in Fig. 7 in which the prongs 25A, 25B are positioned outside the cannula element 12 and the retinal prosthesis 200 may be delivered outside the insertion device 100 into the eye of the patient and in many intermediate configurations as the intermediate configuration W" shown in Fig. 8.

The retinal prosthesis 200 is not shown in figs. 7-9 for clarity sake.

In the extended configuration W' the protrusions 52, 53 abuts against the corresponding stop element 50A, 51A so that a further movement of the translation device 15 towards the outside of the cannula element is avoided.

In this way, is it avoided that the translation device 15 emerges more than necessary or desired form the cannula element 12. This allows the eye of the patient to be protected avoiding that the prongs 25A, 25B touch the walls of the eye 300 damaging them.

In the extended configuration W' the prongs 25A, 25B are positioned outside of the cannula element 12, the body 16 of the translation device 15 being positioned inside the handling portion 3.

As the prongs 25A, 25B are positioned outside the cannula element 12, they can be elastically deformed for delivering the retinal prosthesis 200 into the eye 300 of the patient. The prongs 25A, 25B hold the retinal prosthesis 200 during its movement along the longitudinal cavity 5 and any relative movement between the holding portion 3 and the retinal prosthesis 200 is avoided.

The retinal prosthesis 200 is carried by the prongs 25A, 25B along the longitudinal cavity 5 outside the insertion device 100. Therefore, the retinal prosthesis 200 is not damaged or deformed in the translation along the insertion device 100.

This assures that the retinal prosthesis is delivered in the correct position into the eye of a patient, and also that the retinal prosthesis 200 remain in the coiled configuration P.

The translation device 15 is internally hollow so as to define a safety hole 15A extending along the length of the translation device 15 in which a pushing element 75, shown in Fig. 9B, could be inserted for pushing the retinal prosthesis 200 in the coiled configuration P out from the holding portion 3, as better explained in the followings.

In use, the safety hole 15A is coaxial with the longitudinal hole 24 and with the trough hole 8A so that by inserting the pushing element 75 in the insertion device 100 through the through hole 8A, the retinal prosthesis 200 held by the prongs 25A, 25B can be reached and pushed.

In use, the surgeon firstly prepares the patient's eye 300 and performs a corneal or a scleral incision 70.

For example, as visible in Fig. 16, an incision 70 is performed, having a width 71 of approximately 7 mm, a length 72 of about 4 mm and is positioned at a radial distance 73 of about 6 mm from the eye limbus. Following the incision, a vitrectomy procedure is performed to remove the vitreous from the eye.

For using the insertion device 100, the translation device 15 is inserted into the longitudinal cavity 5 and the nut 21 is actuated so as to translate the translation device 15 along the longitudinal axis X of the longitudinal cavity 5 to the extended configuration W'.

The translation device 15 is inserted so that the threaded portion 17 is positioned in correspondence of the nut 21 and the protrusions 52-53 of the guide portion 18 are inserted in the grooves 50-51 provided in the longitudinal cavity 5.

As the external thread 17A of the threaded portion 17 shapingly couples with the internal thread 23 defined in the nut 21, rotating the nut 21 the translation device 15 is actuated.

The rotation of the nut 21 would cause a corresponding rototranslation of the translation device 15, as the nut 21 cannot move axially in relation to the actuating cavity 9. Nonetheless, the provision of the guide device of the insertion device does not allow the rotation of the translation device 15 in the longitudinal cavity 5, as the protrusions 52-53 are inserted into the grooves 50-51 and can slide along the grooves 50-51 in the longitudinal direction but are unable to exit from the grooves 50-51.

Therefore, rotating the nut 21, the translation device 15 is translated along the longitudinal cavity 5.

The rotation movement of the actuating mechanism 2 is then converted in a translation movement for translating the translation device 25 and then the retinal prosthesis 200 in relation to the cannula element 12 in the coiled configuration P.

Therefore, the holding portion 3 is translated along the longitudinal axis X of the longitudinal cavity 5.

With the insertion device 100 in the extended configuration W', i.e. with the prongs 25A, 25B positioned outside from the cannula element 12, the retinal prosthesis 200 in the coiled configuration P is positioned in the holding portion 3 of the insertion device 100 so as to be interposed between and held by the prongs 25A, 25B.

Being the prongs 25A, 25B, made of flexible material, the insertion of the retinal prosthesis 200 between the prongs 25A, 25B is facilitated and, at the same time, undesired damages to the retinal prosthesis 200 are avoided.

Then the nut 21 is rotated to move the translation device 15 along the longitudinal cavity 5 and to withdraw the prongs 25A, 25B carrying the retinal prosthesis 200 inside the cannula element 12 so that the holding portion 3 is carried in the retracted configuration W.

During this movement the prongs 25A, 25B slide along the wall 5A of the longitudinal cavity 5 and, owing to the configuration of the cannula element 12, the two prongs 25A, 25B cannot open so as to allow the retinal prosthesis to deploy.

The wall 5A of the longitudinal cavity 5 exerts a force on the prongs 25A, 25B which in turn exert a force on the retinal prosthesis 200 keeping the retinal prosthesis in the coiled configuration P.

When the holding portion 3 is in the retracted configuration W, and the prongs 25A, 25B do not protrude outside the cannula element 12, the insertion device 100 can be inserted into the incision 70 in the eye 300.

When the insertion device 100 is correctly positioned in the incision of the eye, for example as indicated in Fig. 17, the nut 21 is rotated by the user so as to move the translation device 15 towards the extended configuration W'.

By rotating the nut 21, the retinal prosthesis 200 is then carried by the prongs 25A, 25B along the longitudinal cavity 5 outside the cannula 12.

This allows for a great reduction in the damage for the retinal prosthesis 200. During the translation of the translation device 15 the prongs 25A, 25B slide along the wall 5A of the cavity, being the prongs 25A, 25B interposed between the retinal prosthesis 200 and the wall 5A, the prongs 25A, 25B protect the retinal prosthesis 200 from being damaged.

The retinal prosthesis 200 is not subjected to any friction or relative movement in the translation of the retinal prosthesis 200 in the longitudinal cavity 5.

Additionally, the dimension of the longitudinal cavity 5 avoids any undesired deployment of the retinal prosthesis 20 in the insertion device 100.

Since the prongs 25A, 25B slide on the wall 5A of the longitudinal cavity 5 there is no space for deployment of the prongs 25A, 25B, i.e. no space for uncoiling of the retinal prosthesis 200. The translation device 15 translates along the cannula element 12, i.e. moves in a linear fashion.

As the translation device 15 does not rotate in the longitudinal cavity 5, the translation device 15 may have any type of external profile, i.e. the section of the translation device 15 may be circular, oval or any custom shape.

It is necessary that the protrusions 52-53 match with the grooves 50-51 so as to be slideably housed in the grooves 50-51 and that the exit of the protrusions 52-53 from the grooves 50-51 is not allowed.

The grooves 50-51 act as guiding rails for the protrusions 52-53 of the translation device 15 preventing it from rotating in the movement of the longitudinal cavity 5. Therefore, when the nut 21 is twisted the holding portion 3 moves up or down along the longitudinal cavity 5 in relation to the cannula 12.

The provision of gripping means on the nut 21 facilitates the actuation by the user.

When the holding portion 3 is translated to the extended configuration W', the prongs 25A, 25B are positioned outside the cannula element 12 and the retinal prosthesis 200 may be delivered into the eye of the patient. The protrusion 52, 53 abut against the stop element 50A-51A so that any further movement of the prongs 25A, 25B outside the cannula element 12 is impeded.

The retinal prosthesis 200 should naturally uncoil overcoming the force exerted by the prongs 25A, 25B, as the two prongs 25A, 25B are very flexible membranes.

Therefore, the retinal prosthesis 200 uncoils and exits form the longitudinal hole 24 defined by the prongs 25A, 25B.

If, for some reason, the retinal prosthesis 200 does not uncoil and the retinal prosthesis 200 is trapped in between the two prongs 25A, 25B, then it is provided for inserting the pushing element 75 at the back of the translating device 15 and to move it into the cavity 5 so as to abut the retinal prosthesis 200 and to push the latter out from the holding portion 3 into the eye.

The pushing element 75, shown in better detail in Fig. 9B, is provided with a shaft body 75A slideable into the cavity 5 and a head 75B having greater dimensions as the shaft body 75A so that the head 75B cannot be housed into the cavity 5.

Therefore, the head 75B of the pushing element 75 acts as a stop element for the movement of the pushing element 75 into the translating device 15.

Therefore, the pushing element 75 can be moved until the distal end 75C of the shaft 75A, opposite to the head 75B is flush with the free ends of the prongs 25A-25B, as visible in Fig. 9A.

Once the retinal prosthesis 200 is injected, the nut 21 is activated to withdraw the translation device 15 inside the handle portion 1, i.e. to move the holding portion in the retracted configuration W, and then the insertion device 100 is removed from the eye 300.

The edges on the insertion device are rounded to minimize damage to the eye if insertion device contacts the eye.

The insertion device is so configured that it can be held in one hand: the thumb and second finger can be used to rotate the nut 21 and the rest of the hand to hold the handle portion 1 of the injection device 100.

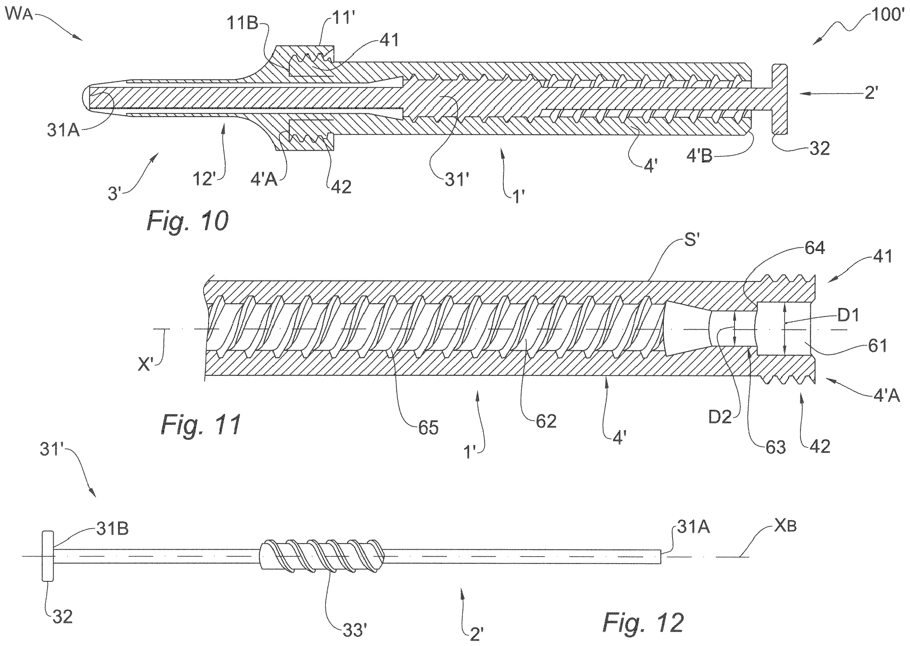

With reference to Figs. 10-15 it is shown, a further version of the invention of the insertion device 100' of the invention. Parts corresponding to the parts already described with reference to the version of Figs. 1-9 will be indicated with the corresponding reference numbers and will not be described in further detail in the followings.

The insertion device 100' comprises a handle portion 1', shown in better detail in Fig. 11, a holding portion 3' shown in better detail in Figs. 13-15 and an actuating mechanism 2', shown in better detail in Fig. 12.

The handle portion 1', the holding portion 3' and the actuating mechanism 2' are connectable to define an insertion device 100' extending along a longitudinal axis X'.

The insertion device 100' is particularly suitable for inserting a retinal prosthesis having dimension bigger than usual retinal prostheses, in particular, it is configured for inserting retinal prosthesis having a length more than 7 mm.

The holding portion 3' and the handle portion 1' are made of transparent material for allowing the surgeon to check the functioning of the insertion device 100' and/or the positioning and the advancement of the retinal prosthesis 200 into the insertion device 100'.

The holding portion 3' comprises a base 11' having a cylindrical shape and provided on its internal surface 11A with a coupling thread 1A for coupling the holding portion 3' to the handle portion 1', as described in the followings.

The holding portion 3' further comprises a cannula element 12' extending from the base 11 and having a cylindrical shape, the cannula element 12' is internally hollow.

The cannula element 12' is provided with a mouth 14' through which the retinal prosthesis 200 is injected into the eye, that is analogously as for the previously described embodiment bevelled, and has a flute-tip.

The holding portion 3' further comprises a tubular element 25' that is internally hollow so as to define an internal longitudinal hole 24' in which the retinal prosthesis 200 in the coiled configuration P is inserted and housed, as shown in Fig. 13, and as will be better explained in the followings.

The tubular element 25' acts as holding element of the insertion device 100'.

The longitudinal hole 24' is cylindrical and extends along a longitudinal axis coaxial with and preferably corresponding to the longitudinal axis X' of the cannula element 12'.

Also the tubular element 25' is coaxial with the cannula element 12'.

The longitudinal hole 24' is filled with lubricant fluid.

The tubular element 25' extends in the holding portion 3' from the base portion 11' up to the cannula element 12'.

The tubular element 25' is positioned in the holding portion 3' so that at least a first portion 25A of the tubular element 25 is arranged inside the base portion 11 and a second portion 25B is arranged inside the cannula element 12'.

The tubular element 25' is so positioned that it is a prolongation of the cannula element 12'. The tubular element 25' is flush with the holding portion once the holding portion 3' is connected and twisted.

The provision of the tubular element 25' allows inter alia plunger 21 to smoothly enter the cannula element 12' and transition from the longitudinal cavity 5' to the cannula element 12'. The tubular element 25' and the base portion 11' are so shaped that an annular chamber 19' is identified between the protruding portion 25A and the inner wall 11A of the base portion 11'.

The configuration of the longitudinal hole 24' avoids any undesired uncoiling or deformation of the retinal prosthesis 200 in the insertion device 100'.

The longitudinal hole 24' is so configured that the pre-coiled retinal prosthesis 200 almost abuts against the wall of the tubular element 25' and undesired movements of the retinal prosthesis 200 or also uncoiling of the retinal prosthesis 200 are avoided.

The retinal prosthesis 200 in the coiled configuration P is inserted into the longitudinal hole 24' so that the coiling axis is parallel with and corresponds with the longitudinal axis X' of the longitudinal hole 24'.

The retinal prosthesis 200 is also so positioned that the spiral 201 of the edge of the retinal prosthesis 200 is perpendicular to the longitudinal axis X' of the longitudinal hole 24' longitudinal hole 24'.

Also in this case there is no space in the longitudinal hole 24' for allowing the uncoiling of the retinal prosthesis 200.

The tubular element 25' has a reduced thickness so as to maximize the dimension of the cross-section of the longitudinal hole 24', so that the cross-section area is mainly hollow and, therefore, available for housing the retinal prosthesis 200 in the coiled configuration P. Reducing the thickness of the different parts of the holding portion 3' allows the encumbrance of the holding portion 3' to be reduced, thus minimizing the invasiveness of the insertion device 100' of the invention and also to maximize the space suitable for housing the retinal prosthesis 200 in the coiled configuration P.

Advantageously, the tubular element 25' has a thickness comprised between 100 µm and 250µm, the longitudinal hole 24' has a diameter comprised between 2mm and 5mm.

The handle portion 1' is arranged to be grasped and held by the surgeon for manoeuvring the insertion device 100' of the invention for injecting a retinal prosthesis 200 into the eye 300 of the patient.

The handle portion 1' comprises a tubular body 4' delimited by an external surface S' extending along a longitudinal axis X' between a first end 4'A and an opposite second end 4'B. The tubular body 4' is internally hollow so as to define a longitudinal cavity 5' extending along the longitudinal axis X' of the tubular body 4' between the opposite first and second ends 4'A, 4'B.

At the first end 4'A, a connecting portion 41 of the tubular body 4' is provided with an external coupling thread 42 arranged on the external surface S' of the tubular body 4'.

The external connecting thread 42 is configured to be coupled with the coupling thread 1A of the holding portion 3' and to cooperate with the latter coupling thread 1A to firmly mutually couple the handle portion 1' and the holding portion 3'.

By screwing the holding portion 3' and the handle portion 1' by means of the external and internal coupling thread 42 and 1A, the handle portion 1' and the holding portion 3' are stably coupled together.

The longitudinal cavity 5' comprises a first cavity part 61 having a diameter D1 so configured to accommodate the tubular element 25', a threaded part 62 arranged for coupling the handle portion 1' and the actuating mechanism 2' for inserting the retinal prosthesis 200 into the eye 300, as will be better explained in the following.

Between the first cavity part 61 and the threaded part 62, the longitudinal cavity 5' is provided with a bridge portion 63 having a diameter D2 lower than the diameter D1 of the first cavity part 61.

The dimensions of the tubular element 25' are so chosen that the tubular element 25' can be housed in the first cavity part 61 but it cannot be housed into the bridge portion 63.

Between the first cavity part 61 and the bridge portion 63 it is provided a resting wall 64 at which, in use, the tubular element 25' abuts when the holding portion 3' and the handle portion 1' are mutually screwed.

When the holding portion 3' and the handle portion 1' are screwed together, the tubular element 25' abuts against the resting wall 64 and the connecting portion 41 of the tubular body 4' is housed into the annular chamber 19' of the holding portion 3' and the first end 4'A abuts against the bottom wall 11B of the base 11', as shown in Fig. 10.

The threaded part 62 is arranged for being coupled with the actuating mechanism 2' so as to allow the actuating mechanism 2' to be advanced longitudinally into the longitudinal cavity 5', as explained in the followings.

The actuating mechanism 2' allows the surgeon to progressively, and in a controlled manner, push the retinal prosthesis 200 in the coiled configuration P from the longitudinal hole 24' inside the eye 300 by means of a twisting motion.

The actuating mechanism 2' comprises a shaft 31' having a substantial cylindrical shape and extending along a longitudinal axis XB that in use corresponds with the longitudinal axis X' of the longitudinal cavity 5' between opposite first and second longitudinal ends 31A, 31B, and a handle 32' arranged ad the second longitudinal end 31B of the shaft 31'.

The shaft 31' is dimensioned so as to be insertable in the longitudinal cavity 5' of the handle portion 1' so as to match with the threaded part 62.

The handle 32' is so dimensioned that it cannot be inserted in the longitudinal cavity 5' of the handle portion 1'.

The handle 32' does therefore act as a stop element for the insertion of the actuating mechanism 2' in the handle portion 1'.

The end of the threaded part 62 of the handle portion 1' acts as stop element, at the end of the threading, the actuating mechanism 2' element cannot be twisted anymore.

The shaft 31' is provided with an advancing thread 33' arranged for being coupled with the threaded part 62 for advancing the actuating mechanism 2' longitudinally into the longitudinal cavity 5'.

The advancing thread 33' is advantageously a double thread for maximising the advancing action of the actuating mechanism 2' in the longitudinal cavity 5'.

The double start threading allows to have a bigger lead for similar mechanical properties than of a single start.

This allows for the surgeon to reduce the number of rotations necessary for pushing the prosthesis into the eye. This increases the effectiveness of the insertion device 100' of the invention reducing the number of twists required by the user for advancing the retinal prosthesis 200 therethrough.

This also increases the safety of the insertion device 100' of the invention reducing the risk of damaging the retinal prosthesis 200, as explained in the followings.

The actuating mechanism 2' is arranged for being inserted in the longitudinal cavity 5' and to be moved in the latter between an retracted configuration in which the first longitudinal end 31A of the shaft 31' is distanced from the retinal prosthesis 200 and an injecting configuration Wa, visible in Fig. 10, in which the first longitudinal end 31A protrudes into the mouth 14' of the cannula element 12' and the retinal prosthesis 200 is injected into the eye 300 of the patient.

The first longitudinal end 31A cannot protrude further than the mouth 14 to avoid damages to the eye.

The actuating mechanism 2' and the handle portion 1' are so dimensioned that when the actuating mechanism 2' is inserted into the longitudinal cavity 5' the first longitudinal end 31A is distanced form the retinal prosthesis 200 housed in the longitudinal hole 24', as better explained in the followings.

This allows possible damages to the retinal prosthesis 200 to be avoided.

This also allows that the retinal prosthesis 200 is advanced as a consequence of a twisting action.

Any undesired movement and/or deformation of the retinal prosthesis 200 is also avoided. This greatly increases the positioning precision obtainable with the device of the invention.

This allows the retinal prosthesis 200 to be protected and to avoid any damage thereto. In other words, the retinal prosthesis 200 can be advanced along the tubular element 25' only as a consequence of the twisting action exerted by the surgeon on the actuating mechanism 2', i.e. on the handle 32'.

This increases the precision of the insertion device 100' of the invention compared with known devices.

Additionally, the actuating mechanism 2' is so shaped that when the actuating mechanism 2' is advanced along the longitudinal cavity 5', the longitudinal end 31A extends to the mouth 14' of the cannula element 12' and protrudes into mouth 14' up to the wall 13'.

The different elements of the insertion device 100' of the invention, i.e. the holding portion 3', the handle portion 1' and the actuating mechanism 2', are so dimensioned to minimise the overall dimension of the insertion device and to reduce the invasiveness of the device.

It is also provided for configuring the holding portion 3', the handle portion 1' and the actuating mechanism 2', so as to eliminate any play between the different elements and to minimize numbers of twist the surgeon has to perform for inserting the retinal prosthesis 200 into the eye 300.

In use, the surgeon prepares the eye making the incision 305 and performs a vitrectomy to remove the vitreous from the eye.

Then a large pre-coiled retinal prosthesis 200 is inserted in the coiled configuration into the longitudinal hole 24' of the holding portion 3', previously filled with a lubricant, as shown in Fig. 13.

The retinal prosthesis 200 in the coiled configuration P is so positioned that the coiling axis Xc is parallel with the longitudinal axes X' of the tubular element 25'.

The holding portion 3' is then connected with the handle portion 1' by screwing the external coupling thread 23 on the coupling thread 1A so that the connecting portion 41 of the tubular body 4' is housed into the annular chamber 19'.

Then the actuating mechanism 2' is inserted into the longitudinal cavity 5' of the handle portion 1' through the second end 4B of the tubular body 4' and rotated to couple the advancing thread 33' and threaded part 27' together to firmly coupling the handle portion 1' and the holding portion 3'.

Then the cannula element 12' is inserted into the incision 70, as visible in Fig. 17, so that the wall 13' faces the lens 301 of the eye and it is closest and in a tangent plane to the lens 301, and the mouth 14' faces the retina 302 of the eye 300. This orientation will protect the lens 301 and ensure that the retinal prosthesis 200 unfolds in the direction of the retina 302, opposite to the lens 301.

After that, the shaft 31 is rotated for advancing the longitudinal end 31A along the longitudinal cavity 5' towards the retinal prosthesis 200 and for pushing the retinal prosthesis 200 outside from the cannula element 12'.

As the shaft 31 is rotated, the longitudinal end 31A is advanced towards the retinal prosthesis 200 and brought into contact with it.

The longitudinal end 31A contacts almost all the turnings of the spiral 201 and by continuing to screw the shaft 31, the longitudinal end 31A pushes the retinal prosthesis 200 along the tubular element 25' as indicated by the arrow F1A in Fig. 13.

Usually, approximately five turns are necessary for ensuring the ejection of the retinal prosthesis 200 into the eye 300.

The extension of the threaded part 62 of the handle portion 1' avoids an excessive progression of the shaft 31 past the wall 13 of the mouth 14.

This will ensure that the surgeon does not damage any structure of the eye.

The retinal prosthesis 200 is then ejected from the cannula element 12' and delivered into the eye 300.

When the retinal prosthesis 200 is outside from the cannula element 12' automatically uncoils and configures itself in the uncoiled configuration P1 shown in Fig. 17.

When the retinal prosthesis 200 is ejected from the cannula element 12', it floats in the eyeball ready to be fixed to the retina 302.

Then the insertion device 100' is pulled back out from the incision 70.

The insertion device 100, 100' of the invention is minimally invasive and is suitable for inserting large retinal prostheses 200 into the eye 300 and facilitates the loading of a large pre-coiled retinal prosthesis 200 in the coiled configuration P.

The insertion device 100, 100' enables the insertion through a relatively small incision 70 in the eye of approximately 7 millimeters in length and enables the injection of the retinal prosthesis 200 in a controlled manner.

In addition, the insertion device 100, 100' is designed to minimize the amount of damage that could be inflicted upon the patient's lens during the injection process.

The different elements of the insertion device 100, 100' of the invention, i.e. the holding portion 3' 3, the handle portion 1, 1' and the actuating mechanism 2, 2', are so dimensioned to minimise the overall dimension of the insertion device and to reduce the invasiveness of the device.

The actuating mechanism 2, 2' of the invention is so configured to increase the efficiency of the insertion device of the invention and to increase the positioning precision obtainable and to preserve the retinal prosthesis from any possible damages.

The features of the insertion device 100, 100' also avoid any undesired movement of the retinal prosthesis into the device of the invention.

## Claims

1. Insertion device (100; 100') for inserting a retinal prosthesis (200) into the eye of a patient comprising:
- a handle portion (1; 1') arranged to be grasped by a user of the insertion device (100; 100'),
- a cannula element (12; 12') arranged to be inserted into the eye of a patient positioned at a longitudinal end (4A) of the handle portion (1; 1'),
- a holding portion (3; 3') arranged for holding the retinal prosthesis (200) in the coiled configuration (P),
- the holding portion (3; 3') being provided with an internal longitudinal hole (24; 24') so dimensioned to house the retinal prosthesis (200) in the coiled configuration (P) and to avoid the retinal prosthesis (200) to uncoil in the insertion device (100; 100'),
- an actuating mechanism (2, 21; 2', 32') coupled to the handling portion (1; 1') and arranged for being rotated for moving the retinal prosthesis (200) in relation to the cannula element (12; 12')for inserting the retinal prosthesis (200) into the eye (300) of the patient, wherein the insertion device (100; 100') further comprises a conversion mechanism for transforming the rotation movement of the actuating mechanism (2, 21; 2', 32') in a translation movement for moving the retinal prosthesis (200) in relation to the cannula element (12; 12').

2. Insertion device according to claim 1, wherein the handle portion (1; 1') is provided with a longitudinal cavity (5 ;5') and the actuating mechanism (2, 21; 2', 32') comprises a translating device (15) for translating the holding portion (3; 3') along the longitudinal cavity (5 ;5') in relation to the cannula element (12; 12') for inserting the retinal prosthesis (100) into the eye of the patient so that the retinal prosthesis (200) is carried by the holding portion (3; 3') along the longitudinal cavity (5 ;5') and outside from the insertion device.

3. Insertion device according to claim 1, or 2, wherein the insertion device (100; 100') is provided with stroke limiting elements (50A, 51A) so that the holding portion (3; 3') is slideable in the longitudinal cavity (5 ;5') in relation to the cannula element (12; 12') between a retracted configuration (W) in which the holding portion (3; 3') is positioned inside the cannula element (12; 12') and an extended configuration (W') in which the holding portion (3; 3') is positioned outside the cannula element (12; 12') and the retinal prosthesis (200) may be delivered outside the insertion device into the eye of the patient.

4. Insertion device according to any one of the preceding claims, and further comprises a guide device (50, 51, 52, 53; 18) for guiding the translation of the translation device (15) into the insertion device (100, 100') along the longitudinal cavity (5 ;5').

5. Insertion device according to claim 4, wherein the guide device comprises at least one groove (50, 51) defined in the wall (5A) of the longitudinal cavity (5; 5') and extending along the longitudinal axis (X) of the longitudinal cavity (5; 5') and configured to avoid the rotation of the translation device (15) in its movement along the insertion device about the longitudinal axis (X) of the longitudinal cavity.

6. Insertion device according to claim 5, wherein the guide device further comprises at least one protruding portion (52, 53) arranged to match with the at least one groove (50, 51) so as to be slideably housed in the groove (50, 51) for cooperating with the groove for guiding the movement of the translation device 15 along the longitudinal cavity (5; 5').

7. Insertion device according to claim 6, wherein the guide device comprises a pair of grooves (50, 51) defined at diametrically opposing position in the longitudinal cavity (5 ;5') and a pair or diametrically opposed protrusion (52, 53) in the translation device (15).

8. Insertion device according to any one of claim 4 to 7, wherein the guide device further comprises a stop element (50A, 51A) for limiting the stroke of the translating device (15) in the longitudinal cavity (5 ;5').

9. Insertion device according to any one of the preceding claims, wherein the holding portion (3; 3') is provided with at least two holding elements (25A, 25B) defining a longitudinal hole (24') therebetween, the retinal prosthesis (200) being inserted and held between the holding elements (25A, 25B) in the coiled configuration P.

10. Insertion device according to the preceding claim, wherein the holding elements comprises two prongs (25A, 25B) extending along a longitudinal axis (X) of the holding portion (3) and mutually facing, the retinal prosthesis (200) in the coiled configuration (P) being inserted between the two prongs (25A, 25B) so that the coiling axis (Xc) is substantially parallel to the prongs (25A, 25B).

11. Insertion device according to claim 9 or 10, wherein the prongs (25A, 25B) are made of a flexible material, preferably of plastics such as polypropylene, polycarbonate or Accura 60, so as to be deformable to help the delivery of the retinal prosthesis outside from the insertion device.

12. Insertion device according to claim 1, wherein, the holding portion (3') is fixed in relation to the handle portion (1') and to the cannula element (12'), the device being provided with a pushing element (31) for pushing the retinal prosthesis (200) in the coiled configuration (P) outside the cannula element and for moving the retinal prosthesis (200) in relation to the holding portion (3').

13. Insertion device according to claim 12, wherein the holding portion is defined in a base portion (11') of the cannula element (12'), the base portion (11') being provided with a tubular element (25') arranged within the base portion (11') and the cannula element (12') and defining the holding portion in which the retinal prosthesis in the coiled configuration is inserted and moved.

14. Insertion device according to claim 13, wherein the tubular element (25') is advantageously concentric with the base portion (11') and the cannula element (12').

15. Insertion device according to any one of claims 12 to 14, wherein the base portion (11') is provided with connecting means for being connected to the handle portion (1') so as to removably connecting the cannula element (12') to the handle portion (1') of the insertion device.
